# EUROPEAN PATENT SPECIFICATION

(11) **EP 0 830 341 B1**
(45) Date of publication and mention of the grant of the patent: **05.09.2001**
(21) Application number: 96917222.0
(22) Date of filing: 04.06.1996
(51) Int. Cl.: C07C 319/18, C07C 319/20, C07C 323/22, C07C 323/60

(54) **PROCESSES FOR THE PREPARATION OF 3-(METHYLTHIO)PROPANAL AND 2-HYDROXY-4-(METHYLTHIO)BUTANENITRILE**
VERFAHREN ZUR HERSTELLUNG VON 3-(METHYLTHIO)PROPANAL UND 2-HYDROXY-4-(METHYLTHIO)BUTANSÄURENITRIL
PROCEDES POUR LA PREPARATION DE 3-(METHYLTHIO)PROPANAL ET DE 2-HYDROXY-4-(METHYLTHIO)BUTANENITRILE

(30) Priority: 07.06.1995 US 476356; 29.12.1995 US 581249
(43) Date of publication of application: 25.03.1998
(73) Proprietor: Novus International, Inc., St. Louis, MO 63141 (US)
(72) Inventor: BLACKBURN, Thomas, F., St. Louis, MO 63141 (US); PELLEGRIN, Paul, F., St. Louis, MO 63141 (US); KRANZ, Allen, H., St. Louis, MO 63141 (US)
(74) Representative: Eyles, Christopher Thomas
(86) International application number: US9609060
(87) International publication number: WO9640631

(56) References cited:
- FR-A- 976 673
- GB-A- 1 400 702
- US-A- 2 542 768
- US-A- 2 745 745
- US-A- 4 225 516
- CHEMICAL ABSTRACTS, vol. 53, no. 18, 25 September 1959 Columbus, Ohio, US; abstract no. 16940b, Z. BRZOZOWSKI: "Preparation of beta-methylthiopropionaldehydes" column 16940; XP002012273 & ROCZNIKI CHEMII, vol. 33, 1959, pages 217-220,
- CHEMICAL ABSTRACTS, vol. 82, no. 3, 20 January 1975 Columbus, Ohio, US; abstract no. 16318p, page 454; XP002012378 & JP,A,49 024 890 (KANEGAFUCHI CHEMICAL INDUSTRY) 26 June 1974
- CHEMICAL ABSTRACTS, vol. 83, no. 1, 7 July 1975 Columbus, Ohio, US; abstract no. 9197r, page 763; XP002012379 & JP,A,49 024 046 (ASAHI CHEMICAL INDUSTRY) 20 June 1974

## Description

The present invention relates to catalytic processes for the preparation of 3-(methylthio)propanal (hereinafter "MMP") and 2-hydroxy-4-(methylthio)butanenitrile ("HMBN"). More particularly, the present invention relates to processes for preparing MMP and HMBN using novel addition catalysts.

MMP and HMBN are intermediates for the manufacture of both d,l-methionine and 2-hydroxy-4-(methylthio)butanoic acid ("HMBA"). Methionine is an essential amino acid commonly deficient in grains used in animal feed compositions. HMBA provides a source of methionine, and is widely used as a methionine supplement in animal feed formulations.

MMP is produced by the catalytic reaction between acrolein and methyl mercaptan. In a conventional process for the preparation of MMP, liquid acrolein and methyl mercaptan are introduced into a reactor containing liquid phase MMP and a suitable organic base which acts as an olefin/mercaptan addition reaction catalyst. Reaction takes place in the liquid phase. Conventional organic base catalysts for the reaction between acrolein and methyl mercaptan include amines such as pyridine, hexamethylenetetramine and triethylamine. The olefin/mercaptan addition reaction catalyst is typically combined with an organic acid such as acetic acid to inhibit polymerization of acrolein and improve product yield.

HMBN is subsequently produced by the addition reaction between MMP and hydrogen cyanide in the presence of a suitable addition reaction catalyst, which may include the organic bases used to catalyze the reaction between acrolein and methyl mercaptan. Methionine may be produced by reacting HMBN with excess ammonia under high pressure to produce 2-amino-4-(methylthio)butanenitrile and subsequently hydrolyzing the product using a mineral acid to form methionine. Alternatively, methionine may be produced by reacting MMP with ammonium carbonate to form a hydantoin and subsequently hydrolyzing the hydantoin with a base to form methionine. HMBA may be produced by hydrolyzing HMBN using a mineral acid.

A variety of organic bases have been proposed to catalyze the reaction between acrolein and methyl mercaptan to form MMP and the subsequent addition of hydrogen cyanide to form HMBN.

Z. Brzozowski recommends compounds with an elongated molecule, capable of forming onium compounds for use in catalyzing the reaction between methyl mercaptan and acrolein in the synthesis of MMP and specifically mentions dimethylaniline in "Preparation of β-methylthiopropionaldehyde", Roczniki Chem., vol. 33, pp. 217-20 (1959).

In U.S. Patent No. 2,542,768 (Gresham, et al.), a process for the synthesis of HMBN is disclosed in which methyl mercaptan and acrolein are reacted in the presence of an alkaline, preferably amine, catalyst followed by addition of hydrogen cyanide to the resultant MMP reaction mixture in the presence of the same alkaline catalyst. Specific amine catalysts mentioned include pyridine, piperidine, quinoline, methionine nitrile and triethanolamine.

Pyridine has proven to be an effective addition catalyst used in preparing both MMP and HMBN. However, it would be highly beneficial to identify effective alternative addition reaction catalysts for the preparation of these valuable intermediates.

### SUMMARY OF THE INVENTION

Among the several objects of the present invention are the provision of a process for the preparation of MMP by the catalytic reaction between acrolein and methyl mercaptan; the provision of such a process which provides a high MMP reaction yield; the provision of such a process in which the degradation of MMP and production of high molecular weight by-products is maintained at acceptably low levels; the provision of such a process which can produce high quality MMP that can be used directly, without the need for further purification, in the preparation of methionine or HMBA; the provision of a process for the preparation of HMBN by the catalytic reaction between the MMP reaction product and hydrogen cyanide; the provision of such a process which provides a high HMBN reaction yield; and the provision of such a process in which the catalyst remaining in the MMP reaction product may be further used to catalyze the reaction between MMP and hydrogen cyanide to produce HMBN.

Briefly, therefore, the present invention is directed to a process for the manufacture of MMP. The process comprises reacting methyl mercaptan with acrolein in a reaction zone in the presence of a novel olefin/mercaptan addition reaction catalyst. The novel catalyst comprises at least one organic base selected from the group consisting of triisopropanolamine, tripropylamine, imidazole, benzimidazole, 2-fluoropyridine, 4-dimethylaminopyridine, picoline, pyrazine, N-methyldiphenethylamine, N-ethyl-3,3'-diphenyldipropylamine and trialkylamines having from five to twelve carbon atoms in each of the alkyl substituents bonded to the nitrogen atom.

It has been further discovered that the novel olefin/mercaptan addition reaction catalyst used in catalyzing the reaction between acrolein and methyl mercaptan are also useful in catalyzing the reaction between MMP and hydrogen cyanide to produce HMBN. Thus, the present invention is further directed to a process for the manufacture of HMBN comprising reacting MMP with hydrogen cyanide in the presence of an addition reaction catalyst. The addition reaction catalyst comprises at least one organic base selected from the group consisting of triisopropanolamine, tripropylamine imidazole, benzimidazole, 2-fluoropyridine, 4-dimethylaminopyridine, picoline, pyrazine, N-methyldiphenethylamine, N-ethyl-3,3-diphenyldipropylamine and trialkylamines having from five to twelve carbon atoms in each of the alkyl substituents bonded to the nitrogen atom.

In accordance with another embodiment of the present invention, the novel addition catalyst disclosed herein are used to first catalyze the reaction between methyl mercaptan and acrolein to produce an intermediate reaction product mixture comprising MMP and the novel catalyst. Then, without prior separation of the catalyst from the MMP in the intermediate reaction product mixture, the MMP reaction product is reacted with hydrogen cyanide to produce HMBN.

Other objects and features of this invention will be in part apparent and in part pointed out hereinafter.

### DETAILED DESCRIPTION OF THE PREFERRED EMBODIMENTS

In accordance with the present invention, MMP is produced by the reaction between acrolein and methyl mercaptan in the presence of novel olefin/mercaptan addition catalysts. MMP in the reaction product mixture may then be reacted with hydrogen cyanide to produce HMBN using the novel catalysts present in the MMP reaction product mixture as addition reaction catalyst in the cyanidation reaction.

The catalytic reaction between acrolein and methyl mercaptan to produce MMP is well-known and, in the practice of the present invention, this reaction can be carried out in any suitable fashion without particular limitation to the various process conditions employed. For example, acrolein vapor may be absorbed in a liquid reaction medium containing recycled MMP product. The acrolein absorbed in the liquid reaction medium is reacted with methyl mercaptan in the presence of an olefin/mercaptan addition reaction catalyst within the reaction zone of a suitable reactor. Methyl mercaptan is added to the liquid reaction medium in an amount at least substantially stoichiometrically equivalent to the acrolein on a molar basis. A slight excess of methyl mercaptan may be employed. Preferably, about 1 to about 1.02 moles of methyl mercaptan are introduced into the reaction zone for each mole of acrolein present in the liquid reaction medium. The methyl mercaptan and acrolein can be introduced into the liquid reaction medium either simultaneously or successively. The olefin/mercaptan addition reaction catalyst may be present either completely or partially in the MMP or can be introduced into the liquid reaction medium entirely or partially along with the acrolein and methyl mercaptan.

The temperature of the reaction is desirably maintained within the range from about 30 to about 70 °C. Reaction pressure is not critical and may vary within wide limits. However, in order to simplify the reaction apparatus, it is preferred that the reaction be conducted at about atmospheric pressure or at only moderately reduced or elevated pressure.

The reaction between acrolein and methyl mercaptan may be conducted in either a continuous or batchwise fashion. In a batch process, acrolein vapor or liquid may be added to methyl mercaptan in substantially molar equivalent quantities. Alternatively, acrolein and methyl mercaptan may be simultaneously introduced at substantially stoichiometrically equivalent rates of addition into a liquid reaction medium comprising MMP. The reaction medium for a given batch is conveniently provided for a given batch by leaving a heel of MMP in the reactor from a previous batch. Thus, the batch reactor may be operated in a semi-continuous mode in which the acrolein and methyl mercaptan are introduced at a substantially constant rate over a significant portion of the batch cycle, and the reaction product is periodically withdrawn from the reactor, leaving a heel for the next batch.

Fully continuous processes are described, for example, in Biola U.S. Pat. No. 4,225,516 and Hsu et al. U.S. Pat. No. 5,352,837. As described in Hsu et al., the continuous reaction may be carried out by introducing acrolein vapor and methyl mercaptan into a flowing MMP reaction medium in either a co-current or countercurrent gas/liquid contact zone. Alternatively, the initial reaction may be carried out in a stirred tank reactor having an external cooler through which the reaction mixture is circulated.' If the reaction is not completed in the residence time afforded in the initial gas/liquid contact zone, the MMP reaction medium containing unreacted acrolein and methyl mercaptan is forwarded to a second reactor (e.g., a plug flow reactor or a batch holding tank) for completion of the reaction. Preferably, the reaction temperature of the reaction does not exceed about 70 °C in any of the reaction zones.

Olefin/mercaptan addition catalysts for the commercial production of MMP are preferably evaluated on the basis of several criteria, including (1) conversion and yield of MMP; (2) reaction kinetics; and (3) tendency to catalyze unwanted side reactions which produce high molecular weight by-products and decrease product purity, both during the MMP reaction and during subsequent storage of the MMP reaction product. Furthermore, such catalysts are preferably useful in further catalyzing the reaction between MMP and hydrogen cyanide to produce HMBN so that the MMP reaction product mixture containing the addition catalyst can be directly treated with hydrogen cyanide to produce HMBN, without intervening purification.

It has been discovered that certain organic bases which previously had not been recognized as viable olefin/mercaptan addition reaction catalyst may advantageously be used to catalyze the reaction between acrolein and methyl mercaptan to form MMP. Accordingly, the novel catalyst of the present invention includes at least one organic base selected from certain heterocyclic amines, trialkylamines and other tertiary amines in which at least one of the non-hydrogen substituents bonded to the nitrogen atom of the tertiary amine contains an aryl group. The novel olefin/mercaptan addition reaction catalyst may further comprise triisopropanolamine.

The heterocyclic amines which may be present in the novel olefin/mercaptan addition catalyst of the present invention are selected from the group consisting of imidazole, benzimidazole, 2-fluoropyridine, 4-dimethylaminopyridine, picoline (e.g., 2-picoline, 3-picoline and 4-picoline) and pyrazine.

The trialkylamines which may be present in the olefin/mercaptan addition catalyst of the present invention include tripropylamine as well as trialkylamines having at least five carbon atoms in each of the alkyl substituents bonded to the nitrogen atom. The alkyl substituents may be linear, branched or cyclic. In order to take advantage of generally decreased flammability, toxicity and volatility provided by higher molecular weight trialkylamines and avoid solubility problems in the MMP reaction mixture, each of the alkyl substituents of the trialkylamines present in the olefin/mercaptan addition catalyst contains from five to twelve carbon atoms (e.g., tripentylamine, trihexylamine, triheptylamine, trioctylamine, trinonylamine, tridecylamine, triundecylamine, tridodecylamine, etc.).

The novel olefin/mercaptan addition reaction catalyst of the present invention may further include N-methyldiphenethylamine and N-ethyl-3,3'-diphenyldipropylamine.

Each of the aforementioned amines may suitably be used as olefin/mercaptan addition catalyst in the commercial production of MMP. However, with reference to the catalyst evaluation criteria identified herein, some of these organic bases have demonstrated greater overall performance and effectiveness than others. In accordance with a more preferred embodiment of the present invention, the olefin/mercaptan addition catalyst comprises at least one amine selected from the group consisting of triisopropanolamine, imidazole, benzimidazole, picoline, N-methyldiphenethylamine, N-ethyl-3,3'-diphenyldipropylamine and trialkylamines having from five to twelve carbon atoms in each of the alkyl substituents bonded to the nitrogen atom.

Other organic bases may be used as olefin/mercaptan addition reaction catalyst for use in the preparation of MMP, including poly-4-vinylpyridine, t-octylamine, sodium nicotinamide and 3-fluoropyridine. In addition to these organic base catalysts, certain salts may be used to catalyze the reaction between acrolein and methyl mercaptan, including alkali metal acetates, molybdates and formates, either alone or combined with a crown ether or quaternary ammonium salt to enhance the solubility of the salt anion in the MMP reaction mixture, and salts of ethylenediaminetetraacetic acid. Furthermore, we have examined the use of other compounds, viz., zinc acetate, zinc carbonate, p-toluenesulfonic acid, 4-aminobutyric acid and palladium chloride, as catalysts in the preparation of MMP. However, these other compounds are not particularly useful in catalyzing the reaction between acrolein and methyl mercaptan and, in the case of p-toluenesulfonic acid and palladium chloride, appear to be substantially inert in promoting the MMP reaction.

The olefin/mercaptan addition reaction catalyst should be present in the liquid reaction medium in an amount sufficient to effectively catalyze the reaction between acrolein and methyl mercaptan. For example, in a batch process, the molar ratio of the catalyst to methyl mercaptan charged to the reaction zone is from 0.001 to 0.02, preferably from 0.001 to 0.01, especially from 0.001 to 0.005.

It should be noted that some of the novel olefin/mercaptan addition reaction catalysts disclosed herein (e.g., imidazole and benzimidazole) are solids at typical MMP reaction temperatures. If sufficiently soluble, such solid catalysts may be suitably employed by dissolving the catalyst in the liquid MMP reaction mixture. If the catalyst is insufficiently soluble, a minimal amount of a suitable solvent (e.g., water, organic or inorganic acid) may be added to the reaction mixture as a catalyst solubility aid, or the catalyst may simply be suspended in the reaction mixture. However, in order to avoid formation of a separate aqueous phase and possible adverse effects on the reaction between methyl mercaptan and acrolein, the water content of the MMP reaction mixture is preferably controlled so that it is not more than about 6% by weight, more preferably not more than about 3% by weight and especially not more than about 1.5% by weight. Furthermore, if a solid catalyst is employed, it may be advantageous to first dissolve the catalyst in a suitable solvent to form a liquid catalyst premix in order to facilitate addition of the catalyst to the reaction zone.

The novel olefin/mercaptan addition reaction catalysts described herein are preferably combined with an organic or inorganic acid in the reaction zone. The presence of an acid is believed to moderate the basicity of the organic liquid reaction medium, thereby inhibiting undesirable base-catalyzed side reactions which decrease MMP quality. Moreover, the acid may enhance the solubility of solid catalysts in the liquid MMP reaction mixture. A variety of organic acids may be used, including acetic acid, formic acid, citric acid, short-chain fatty acids and organic sulfo-acids (e.g., trifluoromethanesulfonic acid). Suitable inorganic acids include mineral acids such as sulfuric and phosphoric acid. Due to commercial availability and relatively low cost, acetic acid is preferred. The molar ratio of organic base to acetic acid introduced into the reaction zone is typically from 0.5 to 2.0. Preferably, in order to ensure that base-catalyzed side reactions are sufficiently inhibited, the molar ratio of organic base to acetic acid introduced into the reaction zone is from 0.5 to 1.0. Where one or more of the aforementioned bases is combined in the reaction zone with a mineral acid, the mineral acid is preferably sulfuric acid or phosphoric acid. The molar ratio of the organic base to the mineral acid introduced into the reaction zone is preferably from 1 to 50. When one of the organic bases disclosed herein is combined with an organic or inorganic acid in the reaction zone, the liquid reaction medium preferably contains between 0.2% and 0.75% by weight of the organic base/acid combination. In order to simplify addition of the organic base/acid combination to the reaction zone, the catalyst may first be combined with an organic or inorganic acid to form a liquid catalyst premix which is then added to the reaction zone.

MMP reaction product may be used directly for the preparation of HMBN without prior distillation for removal of either high boiling or low boiling impurities. This not only saves the capital and operating expense of distillation, but also avoids the yield losses inevitably resulting from the formation of additional high boilers in an MMP distillation column. HMBN may be produced by reacting the MMP reaction product with hydrogen cyanide in the presence of a suitable addition reaction catalyst. Advantageously, it has been discovered that triisopropanolamine, nicotinamide, imidazole, benzimidazole, 2-fluoropyridine, poly-4-vinylpyridine, 4-dimethylaminopyridine, picoline and pyrazine may serve as addition reaction catalyst in the production of HMBN. Furthermore, trialkylamines having from three to eighteen carbon atoms in each of the alkyl substituents bonded to the nitrogen atom and tertiary amines in which at least one of the non-hydrogen substituents bonded to the nitrogen atom contains an aryl group as disclosed hereinabove may also be used to catalyze the reaction between MMP and hydrogen cyanide to produce HMBN.

Thus, in accordance with a preferred embodiment of the present invention, it is possible to first prepare MMP by reacting methyl mercaptan with acrolein in a reaction zone in the presence of one of the olefin/mercaptan addition reaction catalysts disclosed herein, either alone or in combination with a suitable organic or inorganic acid, to produce an intermediate reaction product mixture containing MMP and the catalyst. Thereafter, and without prior separation of the catalyst from the MMP in the intermediate reaction-product mixture, the MMP can be directly converted to HMBN by reacting the MMP with hydrogen cyanide. In the case where the olefin/mercaptan addition reaction catalyst comprises tripropylamine or a trialkylamine having from five to twelve carbon atoms in each of the alkyl substituents bonded to the nitrogen atom, N-methyldiphenethylamine or 3,3'-diphenyldipropylamine, it is preferred to substantially immediately convert MMP in the intermediate reaction product mixture to HMBN in order to produce HMBN of high quality and in high yield.

The catalytic reaction between MMP and hydrogen cyanide to produce HMBN is well-known and, in the practice of the present invention, this reaction can be carried out in any suitable fashion without particular limitation to the various process conditions employed. The MMP product may be reacted with hydrogen cyanide in either a continuous or batchwise reaction system. Preferably, hydrogen cyanide is present in a slight molar excess of about 2% relative to MMP. The temperature of the cyanidation reaction is desirably maintained within the range from about 30 to about 70 °C, preferably from about 50 to about 70 °C. As in the MMP reaction, the pressure maintained during the cyanidation reaction is not critical and may vary within wide limits, but preferably is close to atmospheric pressure.

Due to their overall effectiveness as catalysts for both the olefin/mercaptan addition reaction and the reaction between MMP and hydrogen cyanide, the addition catalyst used to prepare HMBN in this fashion preferably comprises at least one amine selected from the group consisting of triisopropanolamine, imidazole, benzimidazole, picoline, N-methyldiphenethylamine, N-ethyl-3,3'-diphenyldipropylamine and trialkylamines having from five to twelve carbon atoms in each of the alkyl substituents bonded to the nitrogen atom.

The MMP and hydrogen cyanide must be reacted in the presence of a sufficient amount of addition catalyst to effectively promote the cyanidation reaction. For some catalyst systems, a greater quantity of addition catalyst may be employed during the cyanidation reaction than is present during the reaction between acrolein and methyl mercaptan. Thus, an excess of addition catalyst may be used initially during the MMP reaction in order to insure that a sufficient quantity of catalyst is present in the intermediate reaction product mixture to effectively catalyze the reaction between MMP and hydrogen cyanide. However, using an excess of addition catalyst in the reaction between acrolein and methyl mercaptan to later achieve optimal hydrogen cyanide addition may cause excessive degradation of the MMP reaction product. In such cases, it is preferred that an additional amount of an organic base catalyst be introduced into the intermediate reaction product mixture immediately prior to the introduction of hydrogen cyanide to further promote the cyanidation reaction. The catalyst added to the intermediate reaction product mixture may be selected from any of the addition catalysts disclosed herein and may, in fact, be the same catalyst used to catalyze the reaction between acrolein and methyl mercaptan. Alternatively the added catalyst may comprise a conventional organic base catalyst (e.g., pyridine, triethylamine, hexamethylenetetramine etc.). Preferably, prior to the introduction of the additional catalyst, the concentration of the addition reaction catalyst in the intermediate reaction product mixture is between about 0.01% and about 1% by weight, more preferably between about 0.05% and about 0.25% by weight, and after the further amount of catalyst is introduced into the intermediate reaction product mixture, the intermediate reaction product mixture contains between about 0.05% and about 1% by weight, more preferably between about 0.1% and about 0.5% by weight of addition catalyst.

The HMBN produced by the process of the present invention may be directly converted without purification to HMBA by either the process described in Ruest et al. U.S. Pat. No. 4,524,077 or the process of Hernandez U.S. Pat. No. 4,912,257. In the process of the Ruest patent, HMBN is hydrolyzed in sulfuric acid, the HMBA product is extracted from the hydrolyzate using a substantially water-immiscible solvent, and the extract is steam-distilled to produce an 85% to 90% by weight aqueous solution of HMBA. In the process of the Hernandez patent, the hydrolyzate is neutralized with ammonia, causing it to separate into two phases, the organic phase being evaporated and filtered to produce an 85% to 90% by weight aqueous solution of HMBA.

The present invention is illustrated by the following Examples which are merely for the purpose of illustration and are not to be regarded as limiting the scope of the invention or manner in which it may be practiced.

### EXAMPLE 1

The following procedure was used in this Example to assess performance of proposed olefin/mercaptan catalysts for the reaction between acrolein and methyl mercaptan to produce MMP.

The catalyst to be tested was mixed with acrolein and a quantity of this mixture was combined with an excess of methyl mercaptan in a 2 ml reaction vial with septum cap. Methyl mercaptan was transferred using dry ice cooling of both the mercaptan vial and the reaction vial. An excess of methyl mercaptan of about 5% to about 15% by weight based on acrolein was employed. The amount of catalyst present in the reaction vial was chosen to provide approximately 0.0033 moles of catalyst per mole of acrolein or MMP product. In some tests, the catalyst was first combined with an organic or inorganic acid in a molar ratio of approximately 0.7 (catalyst to acid) and this catalyst/acid combination was then added to the acrolein. Also, water was sometimes added to the mixture in the reaction vial to improve catalyst solubility. Where a salt catalyst was employed, it was sometimes combined with a crown ether or a quaternary ammonium salt in a substantially equivalent molar proportion to the salt to improve catalyst solubility.

The reaction vial containing the mixture was held in an oven maintained at about 50 °C. After approximately 30 minutes, the reaction vial was removed and reweighed to determine weight loss during heating (normally less than about 0.002 g). Samples of the reaction mixture contained in the vial were analyzed by gas chromatography for assay and to determine the amount of high molecular weight oligomers present in the mixture.

Table 1 contains a summary of the performance of the alternative aldehyde reaction catalysts which were evaluated using the above-described procedure. Control tests using pyridine and pyridine combined with acetic acid are included for purposes of comparison. Criteria for evaluating catalyst performance included acrolein conversion, amounts of high molecular weight oligomers and qualitative assessment of the appearance of the appropriate chromatogram. Ideally, the MMP reaction mixture contained in the vial will show a low acrolein concentration (indicating high conversion to MMP), low amounts of high molecular weight oligomers (indicating minimal side reactions), and a generally flat gas chromatography base line (indicating the absence of other polymers). Poor quality material will have a pronounced broad peak eluting several minutes after the aldehyde peak, which does not always correlate with oligomer levels. Gas chromatography-mass spectroscopy work has shown this broad peak to be aldehyde, indicating other components are breaking down in the analysis to form this peak. Since in this procedure an excess of methyl mercaptan was used, aldehyde yield was not considered a meaningful evaluation criterion.

In Table 1, the gas chromatography baseline codes (S), (M) and (U) indicate satisfactory, marginal and unsatisfactory, respectively. ND and tr indicate "not detected" and "trace", respectively. All values are reported as weight percent.

The proposed catalysts marked with an asterix are comparative examples.

**Table 1**

| MMP Catalyst (GC assay Baseline Code) | Acrolein | High Molecular Weight Oligomers |
|---|---|---|
| * Pyridine (S) | 0.5 | 9.6 |
| * Pyridine/acetic acid (S) | 0.1 | 0.1 |
| | | |
| Imidazole/acetic acid (S) | tr | 1.1 |
| Imidazole/acetic acid (S) | 0.3 | 1.8 |
| | | |
| Benzimidazole/water/acetic | | |
| acid (S) | 0.2 | 1.0 |
| | | |
| * Nicotinamide (U) | 0.3 | 1.7 |
| * Nicotinamide (U) | 0.9 | 6.5 |
| * Nicotinamide (U) | 0.4 | 3.0 |
| * Nicotinamide/water (U) | ND | 0.2 |
| * Nicotinamide/water (U) | 0.3 | 5.0 |
| * Nicotinamide/acetic acid (S) | 5.2 | |
| * Nicotinamide/acetic acid/water (S) | 0.7 | 2.8 |
| * Nicotinamide/acetic acid/water (S) | ND | 4.0 |
| * Nicotinamide/water/sulfuric acid (U) | 1.6 | tr |
| * Nicotinamide/water/sulfuric acid (U) | ND | 5.0 |
| *Nicotinamide/water/sulfuric acid (U) | 2.8 | < 0.1 |
| * Nicotinamide/water/ phosphoric acid (U) | 0.5 | 4.4 |
| | | |
| * Sodium nicotinamide (U) | | 14.6 |
| * Sodium nicotinamide/acetic acid (U) | ND | 4.9 |
| | | |
| * Poly-4-vinylpyridine (U) | | 8.1 |
| * Poly-4-vinylpyridine/ acetic acid (U) | 0.1 | 1.2 |
| * Poly-4-vinylpyridine/ acetic acid (U) | 0.1 | 2.2 |
| * Poly-4-vinylpyridine/ acetic acid (U) | 0.1 | |
| | | |
| * Sodium acetate/15-crown-5 (M) | 2.0 | 24.8 |
| | | |
| * Sodium acetate/ trioctylmethylammonium chloride (U) | 1.3 | 4.4 |
| | | |
| * Sodium molybdate (U) | 0.1 | 4.7 |
| * Sodium molybdate/acetic | | |
| acid (U) | 0.3 | 7.5 |
| | | |
| * Sodium Formate (U) | < 0.1 | 0.3 |
| | | |
| * Disodium EDTA (U) | 0.1 | 1.3 |
| * Disodium EDTA/acetic acid (U) | ND | 2.3 |
| | | |
| * Palladium chloride (U) | ND | 0.3 |
| | | |
| * p-Toluenesulfonic acid (U) | ND | 5.8 |
| | | |
| * 4-Aminobutyric acid (U) | ND | 2.3 |
| * 4-Aminobutyric acid/ acetic acid (U) | ND | 2.8 |
| * 4-Aminobutyric acid/acetic acid/water (U) | ND | 2.7 |
| | | |
| 2-Fluoropyridine/ acetic acid (S) | 0.1 | < 0.1 |
| | | |
| * 3-Fluoropyridine/ acetic acid (U) | 0.2 | < 0.1 |
| | | |
| Tripropylamine/acetic acid/ water (U) | < 0.1 | < 0.1 |
| | | |
| * Tributylamine/acetic acid (U) | < 0.1 | 0.5 |
| | | |
| * Triphenylamine (U) | < 0.1 | < 0.1 |
| * Triphenylamine/acetic acid (U) | < 0.1 | 0.2 |
| | | |
| * Tribenzylamine (U) | 0.2 | < 0.1 |
| | | |
| Pyrazine/acetic acid (U) | < 0.1 | 0.1 |
| | | |
| * t-Octylamine/acetic acid (U) | 0.2 | 2.0 |
| | | |
| 4-Dimethylaminopyridine/ acetic acid (S) | < 0.1 | 0.5 |
| 4-Dimethylaminopyridine/ acetic acid/water (S) | 0.1 | 0.9 |
| | | |
| * Zinc acetate (U) | 0.4 | 0.4 |
| | | |
| * Zinc carbonate (U) | 0.3 | < 0.1 |

### EXAMPLE 2

The following procedure was used in this Example to assess performance of triisopropanolamine and certain trialkylamines and phenyl group-containing tertiary amines as olefin/mercaptan catalysts for the reaction between acrolein and methyl mercaptan to produce MMP.

In all tests, the catalyst to be tested was first combined with acetic acid in a molar ratio of approximately 0.7. However, in the case of tripropylamine, additional acetic acid was added (molar ratio of catalyst to acetic acid of 0.54) to achieve a single liquid phase. The catalyst/acetic acid combination was mixed with acrolein and a quantity of this mixture was combined with an excess of methyl mercaptan in a 2 ml reaction vial with septum cap. Methyl mercaptan was transferred using dry ice cooling of both the mercaptan vial and the reaction vial. An excess of methyl mercaptan of about 0.4% to about 9% by weight based on acrolein was employed. The amount of catalyst present in the reaction vial was chosen to provide approximately 0.0033 moles of catalyst per mole of acrolein or MMP product.

The reaction vial containing the mixture was held in an oven maintained at about 50 °C. After approximately 30 minutes, the reaction vial was removed and reweighed to determine weight loss during heating (normally less than about 0.002g). Samples of the reaction mixture contained in the vial were analyzed by gas chromatography for assay and to determine the amount of high molecular weight oligomers present in the mixture.

Table 2 contains a summary of performance of this group of alternative aldehyde reaction catalysts which were evaluated using the above-described procedure. The same criteria as set forth in Example 1 were used in the present Example to evaluate catalyst performance.

In Table 2, the gas chromatography baseline codes (S), (M), and (U) indicate satisfactory, marginal, and unsatisfactory, respectively. All values are reported as weight percent.

N-methyldiphenylamine marked with an asterix, is a comparative example.

**Table 2**

| MMP Catalyst (GC assay Baseline Code) | Acrolein | High Molecular Weight Oligomers |
|---|---|---|
| Triisopropanolamine/ acetic acid (S) | 0.1 | 0.1 |
| | | |
| Tripropylamine/acetic acid (S) | 0.1 | < 0.1 |
| Tripentylamine/acetic acid (S) | 0.1 | < 0.1 |
| Trioctylamine/acetic acid (S) | 0.1 | < 0.1 |
| Tridodecylamine/acetic acid (S) | 0.1 | < 0.1 |
| | | |
| N-methyldiphenethylamine/ acetic acid (S) | 0.1 | < 0.1 |
| N-ethyl-3,3'-diphenyl-dipropylamine/acetic acid (S) | 0.1 | 0.3 |
| * N-methyldiphenylamine/ acetic acid (U) | < 0.1 | 0.1 |

### EXAMPLE 3

In this Example, a mixture representative of MMP produced using tripropylamine combined with acetic acid to catalyze the aldehyde reaction was converted to HMBN by reacting it with hydrogen cyanide.

An MMP mixture was prepared by mixing water (0.091 g), distilled MMP (6.91 g) and a tripropylamine/acetic acid catalyst solution (0.008 g) containing 0.54 moles of tripropylamine per mole of acetic acid. Hydrogen cyanide (40 µl, 99.5%) was added to 70 µl of this aldehyde/water/catalyst/acetic acid mixture in a reaction vial, using wet ice cooling during transfer. The reaction vial was then placed in a 50° C oven for 30 minutes. The tripropylamine catalyst present in the MMP mixture was used to catalyze the cyanidation reaction. The vial was then removed from the oven and allowed to cool. A sample of the cooled nitrile reaction product contained in the vial was analyzed by gas chromatography for assay and to determine the amount of high molecular weight oligomers present in the mixture. The sample contained 98.2% nitrile, 0.1% high molecular weight oligomers and 0.03% MMP on a weight basis.

### EXAMPLE 4

In this Example, nicotinamide, imidazole, benzimidazole, 2-fluoropyridine, pyrazine and 4-picoline were tested using the procedure described below to further assess their performance as olefin/mercaptan addition catalysts for the reaction between acrolein and methyl mercaptan.

Distilled acrolein having a typical assay of about 97.3% to about 97.5% by weight acrolein and about 2.5% by weight water was mixed with hydroquinone to provide a mixture having a hydroquinone concentration of about 0.10% to about 0.12% by weight. The mixture of distilled acrolein and hydroquinone was stored at 0 to 5 °C. Distilled methyl mercaptan having a typical assay of about 99.3% to about 99.5% by weight methyl mercaptan was employed.

The aldehyde reaction was conducted in a 1000 ml stainless steel reactor with internal cooling coils for temperature control and an agitator. A quantity of MMP was first prepared by reacting acrolein and methyl mercaptan in the presence of a particular aldehyde catalyst. This MMP was then used as a "heel" in preparing a subsequent batch of MMP using the same catalyst. Typically, several batches of MMP were prepared using the same catalyst and an MMP heel from the preceding batch so that steady state conditions were approached.

To make the MMP heel, methyl mercaptan was charged to the reactor followed by the catalyst using a subsurface feed tube. The reactor and its contents were warmed to ambient temperature at which time the acrolein was fed to the reactor over a period of about 25 minutes at a reaction temperature of about 50 °C. About 1.005 moles of methyl mercaptan and about 0.0033 moles of catalyst were charged to the reactor per mole of acrolein. Except in the case of nicotinamide, the catalyst in the reactor was combined with acetic acid using a molar ratio of about 0.7 (catalyst to acetic acid). The aldehyde reaction was finished by maintaining the process temperature at about 50 °C and agitating the contents of the reactor for about 60 minutes. Once the aldehyde reaction was complete, the reactor and its contents were cooled to 20 to 25 °C over a period of about 10 minutes while continuing agitation.

The catalyst (0.0033 moles per mole of acrolein) and, except in the case of nicotinamide, acetic acid (0.7 catalyst to acetic acid molar ratio) were blended with the MMP heel and charged to the reactor for preparation of additional aldehyde. Methyl mercaptan and acrolein were then fed simultaneously to the reactor with agitation over a period of about 30 minutes at a reaction temperature of about 60 °C. Approximately 1.005 moles of methyl mercaptan per mole of acrolein were charged to the reactor. The aldehyde reaction was finished by maintaining the process temperature at about 60 °C for about 23 minutes. Once the aldehyde reaction was complete, the reactor and its contents were cooled to 20 to 25 °C over a period of about 10 minutes. If necessary, the batch reaction sequence was repeated using a particular catalyst and an MMP heel from the preceding batch until steady state conditions were approached.

A sample of the reaction mixture from the final batch was injected into a gas chromatograph for assay. A sample of this reaction mixture was also subjected to gas chromatography analysis to determine the amounts of high molecular weight oligomers in the mixture. In some cases, limited aging studies of the MMP reaction product were carried out to test storage stability.

Table 3 contains a summary of the performance of the alternative aldehyde reaction catalysts which were evaluated using the above-described procedure. The results in Table 3 include the weight percent of methyl mercaptan, acrolein, MMP and high molecular weight oligomers in the final batch reaction mixture. The results from a control test using a pyridine catalyst combined with acetic acid are included for purposes of comparison, as are the results from the test using nicotinamide as the catalyst.

**Table 3**

| MMP Catalyst | MeSH | Acrolein | MMP | High Molecular Weight Oligomers |
|---|---|---|---|---|
| Pyridine/acetic acid | 0.30 | 0.23 | 97.88 | 1.25 |
| Nicotinamide | 0.94 | 0.5 | 89.74 | 9.47 |
| Imidazole/acetic acid | 0.11 | 0.5 | 97.12 | 1.43 |
| Benzimidazole/acetic acid/water | 0.21 | 0.17 | 97.59 | 1.56 |
| 2-Fluoropyridine/ acetic acid | 0.64 | 0.5 | 96.50 | 2.12 |
| Pyrazine/acetic acid | 0.30 | 0.23 | 97.88 | 2.19 |
| 4-picoline/ acetic acid | 0.21 | 0.38 | 98.02 | 1.14 |

Of the catalysts evaluated in this Example, nicotinamide, pyrazine, and 2-fluoropyridine produced MMP having elevated levels of high molecular weight oligomers.

The MMP produced using imidazole, benzimidazole and 4-picoline as catalysts was tested for storage stability at 45 °C. For purposes of comparison, MMP made using pyridine combined with acetic acid was also aged at 45 °C to assess storage stability. In a typical aging study, 40 g of the aldehyde product were placed in a glass bottle which was then transferred to an oven maintained at 45 °C. Periodically, samples of the product were withdrawn from the bottle and analyzed by gas chromatography for assay. The results from the MMP aging study are summarized in Table 4.

**Table 4**

| MMP Catalyst | Days | MMP Loss (wt %MMP/day) |
|---|---|---|
| Pyridine/acetic acid | 30 | 0.10 |
| Imidazole/acetic acid | 11 | 3.98 |
| Benzimidazole/acetic acid | 11 | 1.05 |
| 4-picoline/acetic acid | 10 | 0.30 |
| 4-picoline/acetic acid | 21 | 0.37 |
| 4-picoline/acetic acid | 31 | 0.37 |

At 45 °C, MMP produced using imidazole, benzimidazole and 4-picoline as olefin/mercaptan addition catalysts showed faster deterioration as compared to aldehyde made using pyridine.

The MMP prepared using imidazole, benzimidazole and 4-picoline combined with acetic acid as olefin/addition reaction catalysts was converted to HMBN by reacting it with hydrogen cyanide using the organic base catalyst remaining in the MMP reaction product mixture to further catalyze the cyanidation reaction. For purposes of comparison, the MMP prepared using pyridine/acetic acid as the catalyst was also converted to HMBN.

The nitrile reaction was conducted in the same 1000 ml stainless steel reactor with agitator and internal cooling coils for temperature control. The MMP charge containing the organic base catalyst was first weighed into the reactor. Hydrogen cyanide (99.5%) was then fed to the reactor with agitation over a period of 26 minutes at a temperature of about 60 °C.
Approximately 1.02 moles of hydrogen cyanide per mole of MMP were charged to the reactor. The nitrile reaction was finished by holding the batch without agitation or cooling for about 20 minutes. Once the nitrile reaction was complete, the reactor and its contents were cooled to 20 to 25 °C over a period of about 10 minutes. A sample of the reaction mixture was subjected to gas chromatographic analysis to determine its assay, MMP and oligomer content. The results from the nitrile conversion are summarized in Table 5.

**Table 5**

| HMBN Catalyst | MMP | HMBN | High Molecular Weight Oligomers |
|---|---|---|---|
| Pyridine/acetic acid | ND | 98.5 | 0.7 |
| Imidazole/acetic acid | tr | 98.3 | 1.2 |
| Benzimidazole/ acetic acid | ND | 94.7 | 1.3 |
| 4-picoline/acetic acid | ND | 99.3 | 0.7 |

### COMPARATIVE EXAMPLE 5

Distilled acrolein and methyl mercaptan were used to prepare an MMP heel and batch after the manner of Example 4. Poly-4-vinylpyridine, supplied by Reilley (Reillex 425) was used as the olefin/mercaptan addition reaction catalyst. Poly-4-vinylpyridine (2.7 g) was charged to the empty reactor. Methyl mercaptan (88.4 g) was then charged to the reactor. The reactor and its contents were warmed to ambient temperature at which time acrolein (100.9 g) was fed to the reactor over a period of about 50 minutes at a reaction temperature of about 50 °C. The aldehyde reaction was finished as described in Example 4. Without further catalyst addition, methyl mercaptan (196.1 g) and acrolein (235.9 g) were then fed simultaneously to the reactor with agitation over a period of about 50 minutes at a reaction temperature of about 50 °C. The aldehyde reaction was finished by maintaining the process temperature at about 50 °C for about 30 minutes. A sample of the reaction mixture from the batch was subjected to gas chromatographic analysis. The analysis showed 89.9% MMP, 0.4% acrolein, 0.8% methyl mercaptan, and 0.02% pyridine on a weight basis.

The MMP product containing the poly-4-vinylpyridine catalyst was converted to the nitrile in the same reactor after the manner of Example 4. Hydrogen cyanide (155.4 g) was fed to the reactor containing MMP (600.0 g) with agitation over a period of about 50 minutes at a temperature of about 50 °C. The nitrile reaction was finished by holding the batch without agitation or cooling for about 30 minutes. Once the nitrile reaction was complete, the reactor and its contents were cooled to 20-25 °C over a period of about 10 minutes. A sample of the nitrile reaction mixture was subjected to gas chromatographic analysis. The analysis showed 72.9% HMBN and 2.6% MMP on a weight basis.

In view of the above, it will be seen that the several objects of the invention are achieved.

## Claims

1. A process for the manufacture of 3-(methylthio)propanal comprising reacting methyl mercaptan with acrolein in a reaction zone in the presence of an olefin/mercaptan addition reaction catalyst, said catalyst comprising at least one organic base selected from the group consisting of triisopropanolamine, tripropylamine, imidazole, benzimidazole , 2-fluoropyridine, 4-dimethylaminopyridine, picoline, pyrazine, N-methyldiphenethylamine, N-ethyl-3,3'-diphenyldipropylamine and trialkylamines having from five to twelve carbon atoms in each of the alkyl substituents bonded to the nitrogen atom.

2. A process as set forth in claim 1, wherein said olefin/mercaptan addition reaction catalyst comprises at least one organic base selected from the group consisting of triisopropanolamine, picoline, N-methyldiphenethylamine, N-ethyl-3,3'-diphenyldipropylamine and trialkylamines having from five to twelve carbon atoms in each of the alkyl substituents bonded to the nitrogen atom.

3. A process as claimed in claim 1, wherein said olefin/mercaptan addition reaction catalyst comprises N-methyldiphenethylamine.

4. A process as claimed in claim 1, wherein said olefin/mercaptan addition reaction catalyst comprises N-ethyl-3,3'-diphenyldipropylamine.

5. A process as set forth in any preceding claim, wherein the molar ratio of organic base to methyl mercaptan introduced into said reaction zone is from 0.001 to 0.02.

6. A process as set forth in any preceding claim, wherein said olefin/mercaptan addition reaction catalyst is combined with an organic acid or a mineral acid in said reaction zone.

7. A process as set forth in claim 6, wherein the catalyst is combined with the organic acid and said organic acid is acetic acid and the molar ratio of said organic base to acetic acid introduced into said reaction zone is from 0.5 to 2.0.

8. A process as set forth in claim 7, wherein said reaction zone contains a liquid reaction medium comprising 3-(methylthio)propanal and said catalyst, said liquid reaction medium containing between 0.2% and 0.75% by weight of said catalyst/organic acid combination.

9. A process as set forth in claim 6, wherein the catalyst is combined with the mineral acid and said mineral acid is selected from the group consisting of sulfuric acid and phosphoric acid and the molar ratio of said olefin/mercaptan addition reaction catalyst to said mineral acid introduced into said reaction zone is from 1 to 50.

10. A process as set forth in claim 9, wherein said reaction zone contains a liquid reaction medium comprising 3-(methylthio)propanal and said catalyst, said liquid reaction medium containing between 0.2% and 0.75% by weight of said catalyst/mineral acid combination.

11. A process for the manufacture of 2-hydroxy-4-(methylthio)butanenitrile comprising:
reacting methyl mercaptan with acrolein in a reaction zone in the presence of an olefin/mercaptan addition reaction catalyst comprising at least one organic base selected from the group consisting of triisopropanolamine, tripropylamine, imidazole, benzimidazole, 2-fluoropyridine, 4-dimethylaminopyridine, picoline, pyrazine, N-methyldiphenethylamine, N-ethyl-3,3'-diphenyldipropylamine and trialkylamines having from five to twelve carbon atoms in each of the alkyl substituents bonded to the nitrogen atom, thereby producing an intermediate reaction product mixture comprising 3-(methylthio)propanal and said catalyst; and without prior separation of said catalyst from the 3-(methylthio)propanal of said intermediate reaction product mixture, reacting said 3-(methylthio)propanal with hydrogen cyanide to produce 2-hydroxy-4-(methylthio)butanenitrile.

12. A process as set forth in claim 11 wherein said olefin/mercaptan addition reaction catalyst comprises at least one organic base selected from the group consisting of triisopropanolamine, picoline, N-methyldiphenethylamine, N-ethyl-3,3'-diphenyldipropylamine and trialkylamines having from five to twelve carbon atoms in each of the alkyl substituents bonded to the nitrogen atom.

13. A process as set forth in claim 12, wherein said olefin/mercaptan addition reaction catalyst is combined with an organic acid in said reaction zone.

14. A process as set forth in claim 13, wherein the organic acid is acetic acid.

15. A process as set forth in any one of claims 11 to 14, wherein an additional amount of an organic base is introduced into said intermediate reaction product mixture to promote the reaction of 3-(methylthio)propanal with hydrogen cyanide.

16. A process as set forth in claim 15, wherein the organic base introduced into the intermediate reaction product mixture comprises at least one organic base selected from the group consisting of triisopropanolamine, picoline, N-methyldiphenethylamine, N-ethyl-3,3'-diphenyldipropylamine and trialkylamines having from five to twelve carbon atoms in each of the alkyl substituents bonded to the nitrogen atom.

17. A process as set forth in claim 15 or claim 16, wherein the organic base introduced into the intermediate reaction product mixture is the same organic base which comprises said olefin/mercaptan addition reaction catalyst used in reacting said methyl mercaptan with said acrolein.

18. A process as set forth in claim 15, wherein the organic base introduced into the intermediate reaction product mixture is pyridine.

19. A process as set forth in any one of claims 15 to 18, wherein prior to the introduction of said additional amount of an organic base, said intermediate reaction product mixture contains between 0.01% and 1% by weight of said organic base, and after said additional amount of an organic base is introduced into said intermediate reaction product mixture, said intermediate reaction product mixture contains between 0.05% and 1% by weight of said organic base.

## Patentansprüche

1. Verfahren zur Herstellung von 3-(Methylthio)propanal, bei dem man Methylmercaptan mit Acrolein in einer Reaktionszone in Gegenwart eines Olefin/Mercaptan-Additionsreaktionskatalysators umsetzt, wobei der Katalysator wenigstens eine organischen Base enthält, die aus der Gruppe ausgewählt ist, die aus Triisopropanolamin, Tripropylamin, Imidazol, Benzimidazol, 2-Fluorpyridin, 4-Dimethylaminopyridin, Picolin, Pyrazin, N-Methyldiphenethylamin, N-Ethyl-3,3'-diphenyldipropylamin und Trialkylaminen mit 5 bis 12 Kohlenstoffatomen in jedem der an das Stickstoffatom gebundenen Alkylsubstituenten besteht.

2. Verfahren nach Anspruch 1, bei dem der Olefin/Mercaptan-Additionsreaktionskatalysator wenigstens eine organische Base aufweist, die aus der Gruppe ausgewählt ist, die aus Triisopropanolamin, Picolin, N-Methyldiphenethylamin, N-Ethyl-3,3'-diphenyldipropylamin und Trialkylaminen mit 5 bis 12 Kohlenstoffatomen in jedem der an das Stickstoffatom gebundenen Alkylsubstituenten besteht.

3. Verfahren nach Anspruch 1, bei dem der Olefin/Mercaptan-Additionsreaktionskatalysator N-Methyldiphenethylamin aufweist.

4. Verfahren nach Anspruch 1, bei dem der Olefin/Mercaptan-Additionsreaktionskatalysator N-Ethyl-3,3'-diphenyldipropylamin aufweist.

5. Verfahren nach einem der vorhergehenden Ansprüche, bei dem das Molverhältnis der organischen Base zu dem in die Reaktionszone eingeführten Methylmercaptan in dem Bereich von 0,001 bis 0,02 liegt.

6. Verfahren nach einem vorhergehenden Anspruch, bei dem der Olefin/Mercaptan-Additionsreaktionskatalysator in der Reaktionszone mit einer organischen Säure oder einer Mineralsäure kombiniert wird.

7. Verfahren nach Anspruch 6, bei dem der Katalysator mit der organischen Säure kombiniert wird und die organische Säure Essigsäure ist und das Molverhältnis der organischen Base zu der in die Reaktionszone eingeführten Essigsäure 0,5 bis 2,0 beträgt.

8. Verfahren nach Anspruch 7, bei dem die Reaktionszone ein flüssiges Reaktionsmedium enthält, das 3-(Methylthio)propanal und den Katalysator aufweist und zwischen 0,2 Gew.-% und 0,75 Gew.-% der genannten Kombination Katalysator/organische Säure enthält.

9. Verfahren nach Anspruch 6, bei dem der Katalysator mit der Mineralsäure kombiniert wird und die Mineralsäure aus der aus Schwefelsäure und Phosphorsäure bestehenden Gruppe ausgewählt wird und das Molverhältnis des Olefin/Mercaptan-Additionsreaktionskatalysators zu der in die Reaktionszone eingeführten Mineralsäure 1 bis 50 ist.

10. Verfahren nach Anspruch 9, bei dem die Reaktionszone ein flüssiges Reaktionsmedium enthält, das 3-(Methylthio)propanal und den Katalysator aufweist und zwischen 0,2 Gew.-% und 0,75 Gew.-% der Kombination Katalysator/Mineralsäure enthält.

11. Verfahren zur Herstellung von 2-Hydroxy-4-(methylthio)butannitril, bei dem man Methylmercaptan mit Acrolein in einer Reaktionszone in Gegenwart eines Olefin/Mercaptan-Additionsreaktionskatalysators umsetzt, der wenigstens eine organische Base aufweist, die aus der Gruppe ausgewählt ist, die aus Triisopropanolamin, Tripropylamin, Imidazol, Benzimidazol, 2-Fluorpyridin, 4-Dimethylaminopyridin, Picolin, Pyrazin, N-Methyldiphenethylamin, N-Ethyl-3,3'-diphenyldipropylamin und Trialkylaminen mit 5 bis 12 Kohlenstoffatomen in jedem der an das Stickstoffatom gebundenen Alkylsubstituenten besteht, wodurch ein 3-(Methylthio)propanal und den genannten Katalysator enthaltendes Reaktionszwischenproduktgemisch gebildet wird, und bei dem man ohne vorherige Abtrennung des Katalysators von dem 3-(Methylthio)propanal des Reaktionszwischenproduktgemisches das 3-(Methylthio)propanol mit Cyanwasserstoff zu 2-Hydroxy-4-(methylthio)butannitril umsetzt.

12. Verfahren nach Anspruch 11, bei dem der Olefin/Mercaptan-Additionsreaktionskatalysator wenigstens eine organische Base aufweist, die aus der Gruppe ausgewählt ist, die aus Triisopropanolamin, Picolin, N-Methyldiphenethylamin, N-Ethyl-3,3'-diphenyldipropylamin und Trialkylaminen mit 5 bis 12 Kohlenstoffatomen in jedem der an das Stickstoffatom gebundenen Alkylsubstituenten besteht.

13. Verfahren nach Anspruch 12, bei dem der Olefin/Mercaptan-Additionsreaktionskatalysator mit einer organische Säure in der Reaktionszone kombiniert wird.

14. Verfahren nach Anspruch 13, bei dem die organische Säure Essigsäure ist.

15. Verfahren nach einem der Ansprüche 11 bis 14, bei dem eine zusätzliche Menge einer organischen Base in das Reaktionszwischenproduktgemisch eingeführt wird, um die Umsetzung von 3-(Methylthio)propanal mit Cyanwasserstoff zu fördern.

16. Verfahren nach Anspruch 15, bei dem die in das Reaktionszwischenproduktgemisch eingeführte organische Base wenigstens eine organische Base aufweist, die aus der Gruppe ausgewählt ist, die aus Triisopropanolamin, Picolin, N-Methyldiphenethylamin, N-Ethyl-3,3'-diphenyldipropylamin und Trialkylaminen mit 5 bis 12 Kohlenstoffatomen in jedem der an das Stickstoffatom gebundenen Alkylsubstituenten besteht.

17. Verfahren nach Anspruch 15 oder 16, bei dem die in das Reaktionszwischenprodukt eingeführte organische Base die gleiche organische Base ist, welche der genannte Olefin/Mercaptan-Additionsreaktionskatalysator aufweist, der zur Umsetzung des Methylmercaptans mit dem Acrolein benutzt wird.

18. Verfahren nach Anspruch 15, bei dem die in das Reaktionszwischenproduktgemisch eingeführte organische Base Pyridin ist.

19. Verfahren nach einem der Ansprüche 15 bis 18, bei dem das Reaktionszwischenproduktgemisch vor Einführung der genannten zusätzlichen Menge einer organischen Base zwischen 0,01 und 1 Gew.-% der organischen Base und nach Einführung der zusätzlichen Mengen einer organischen Base in das Reaktionszwischenproduktgemisch zwischen 0,05 und 1 Gew.-% der organischen Base enthält.

## Revendications

1. Procédé pour la fabrication de 3-(méthylthio)propanal comprenant la réaction de méthylmercaptan avec de l'acroléine dans une zone de réaction en présence d'un catalyseur de réaction d'addition d'oléfine/mercaptan, ledit catalyseur comprenant au moins une base organique choisie parmi la triisopropanolamine, la tripropylamine, l'imidazole, le benzimidazole, la 2-fluoropyridine, la 4-climéthylaminopyridine, la picoline, la pyrazine, la N-méthyldiphénéthylamine, la N-éthyl-3,3'-diphényldipropylamine et des trialkylamines ayant de cinq à douze atomes de carbone dans chacun des substituants alkyle liés à l'atome d'azote.

2. Procédé selon la revendication 1, dans lequel ledit catalyseur de réaction d'addition d'oléfine/mercaptan comprend au moins une base organique choisie parmi la triisopropanolamine, la picoline, la N-méthyldiphénéthylamine, la N-éthyl-3,3'-diphényldipropylamine et des trialkylamines ayant de cinq à douze atomes de carbone dans chacun des substituants alkyle liés à l'atome d'azote.

3. Procédé selon la revendication 1, dans lequel ledit catalyseur de réaction d'addition d'oléfine/mercaptan comprend de la N-méthyldiphénéthylamine.

4. Procédé selon la revendication 1, dans lequel ledit catalyseur de réaction d'addition d'oléfine/mercaptan comprend de la N-éthyl-3,3'-diphényidipropylamine.

5. Procédé selon l'une quelconque des revendications précédentes, dans lequel le rapport molaire de base organique au méthylmercaptan introduit dans ladite zone de réaction est compris entre 0,001 et 0,02.

6. Procédé selon l'une quelconque des revendications précédentes, dans lequel ledit catalyseur de réaction d'addition d'oléfine/mercaptan est combiné avec un acide organique ou avec un acide minéral dans ladite zone de réaction.

7. Procédé selon la revendication 6, dans lequel le catalyseur est combiné avec l'acide organique et ledit acide organique est l'acide acétique et le rapport molaire de ladite base organique à l'acide acétique introduit dans ladite zone de réaction est compris entre 0,5 et 2,0.

8. Procédé selon la revendication 7, dans lequel ladite zone de réaction contient un milieu réactionnel liquide comprenant du 3-(méthylthio)propanal et ledit catalyseur, ledit milieu réactionnel liquide contenant entre 0,2 % et 0,75 % en poids de ladite combinaison de catalyseur/acide organique.

9. Procédé selon la revendication 6, dans lequel le catalyseur est combiné avec l'acide minéral et ledit acide minéral est choisi parmi l'acide sulfurique et l'acide phosphorique et le rapport molaire dudit catalyseur de réaction d'addition d'oléfine/mercaptan audit acide minéral introduit dans ladite zone de réaction est compris entre 1 et 50.

10. Procédé selon la revendication 9, dans lequel ladite zone de réaction contient un milieu réactionnel liquide comprenant du 3-(méthylthio)propanal et ledit catalyseur, ledit milieu réactionnel liquide contenant entre 0,2 % et 0,75 % en poids de ladite combinaison de catalyseur/acide minéral.

11. Procédé pour la fabrication de 2-hydroxy-4-(méthylthio)butanenitrile comprenant :
la réaction de méthylmercaptan avec de l'acroléine dans une zone de réaction en présence d'un catalyseur de réaction d'addition d'oléfine/mercaptan comprenant au moins une base organique choisie parmi la triisopropanolamine, la tripropylamine, l'imidazole, le benzimidazole, la 2-fluoropyridine, la 4-diméthylaminopyridine, la picoline, la pyrazine, la N-méthyldiphénéthylamine, la N-éthyl-3,3'-diphényldipropylamine et des trialkylamines ayant de cinq à douze atomes de carbone dans chacun des substituants alkyle liés à l'atome d'azote, produisant par là un mélange de produits intermédiaires de la réaction comprenant du 3-(méthylthio)propanal et ledit catalyseur; et sans séparation préalable dudit catalyseur du 3-(méthylthio)propanal dudit mélange de produits intermédiaires de la réaction, la réaction dudit 3-(méthylthio)propanal avec du cyanure d'hydrogène pour produire du 2-hydroxy-4-(méthylthio)butanenitrile.

12. Procédé selon la revendication 11, dans lequel ledit catalyseur de réaction d'addition d'oléfine/mercaptan comprend au moins une base organique choisie parmi la triisopropanolamine la picoline, la N-méthyldiphénéthylamine, la N-éthyl-3,3'-diphényldipropylamine, et des trialkylamines ayant de cinq à douze atomes de carbone dans chacun des substituants alkyle liés à l'atome d'azote.

13. Procédé selon la revendication 12, dans lequel ledit catalyseur de réaction d'addition d'oléfine/mercaptan est combiné avec un acide organique dans ladite zone de réaction.

14. Procédé selon la revendication 13, dans lequel l'acide organique est l'acide acétique.

15. Procédé selon l'une quelconque des revendications 11 à 14, dans lequel une quantité supplémentaire d'une base organique est introduite dans ledit mélange des produits intermédiaires de la réaction pour promouvoir la réaction du 3-(méthylthio)propanal avec le cyanure d'hydrogène.

16. Procédé selon la revendication 15, dans lequel la base organique introduite dans le mélange des produits intermédiaires de la réaction comprend au moins une base organique choisie parmi la triisopropanolamine, la picoline, la N-méthyldiphénéthylamine, la N-éthyl-3,3'-diphényldipropylamine et des trialkylamines ayant de cinq à douze atomes de carbone dans chacun des substituants alkyle liés à l'atome d'azote.

17. Procédé selon la revendication 15 ou la revendication 16, dans lequel la base organique introduite dans le mélange des produits intermédiaires de la réaction est la même base organique, qui comprend ledit catalyseur de réaction d'addition d'oléfine/mercaptan, utilisé dans la réaction dudit méthylmercaptan avec ladite acroléine.

18. Procédé selon la revendication 15, dans lequel la base organique introduite dans le mélange des produits intermédiaires de la réaction est la pyridine.

19. Procédé selon l'une quelconque des revendications 15 à 18, dans lequel avant l'introduction de ladite quantité supplémentaire d'une base organique, ledit mélange des produits intermédiaires de la réaction contient entre 0,01 % et 1 % en poids de ladite base organique, et après que ladite quantité supplémentaire d'une base organique est introduite dans ledit mélange des produits intermédiaires de la réaction, ledit mélange des produits intermédiaires de la réaction contient entre 0,05 % et 1 % en poids de ladite base organique.
